# EUROPEAN PATENT APPLICATION

(11) **EP 4 364 748 A1**
(43) Date of publication of application: **08.05.2024**
(21) Application number: 22831964.6
(22) Date of filing: 27.06.2022
(51) Int. Cl.: A61K 36/75, A61K 36/538, A61K 36/484, A61K 36/34, A61K 36/238, A61K 36/237, A61K 36/236, A61K 36/233, A61K 36/232, A61K 36/23, A61K 36/076, A61P 11/06

(54) **USE OF TRADITIONAL CHINESE MEDICINE COMPOSITION IN PREPARATION OF DRUG FOR TREATING COUGH VARIANT ASTHMA**

(30) Priority: 29.06.2021 CN 202110729350
(71) Applicant: Shandong New Time Pharmaceutical Co., Ltd., Feixian County, Shandong Province 273400 (CN)
(72) Inventor: ZHANG, Guimin, Linyi, Shandong 276006 (CN); LI, Xin, Linyi, Shandong 276006 (CN); LIU, Dongguang, Linyi, Shandong 276006 (CN)
(74) Representative: J A Kemp LLP
(86) International application number: PCT/CN2022/101536
(87) International publication number: WO 2023/274150

(57) **Abstract**

A traditional Chinese medicinal composition is prepared from *herba schizonepetae, radix saposhnikoviae,* notopterygium root, *radix angelicae pubescentis, radix bupleuri,* common hogfennel root, *rhizoma chuanxiong, fructus aurantii,* poria cocos, *radix platycodonis,* and liquorice root. The traditional Chinese medicinal composition can reduce the cough frequency of a guinea pig model with cough variant asthma, a percentage of eosinophils in a bronchoalveolar lavage fluid, and a level of serum eosinophil cationic proteins, and has a prevention and treatment effect on the cough variant asthma.

## Description

### TECHNICAL FIELD

The present invention relates to new use of a traditional Chinese medicinal composition, particularly relates to use of a traditional Chinese medicinal composition in the preparation of a drug for treating cough variant asthma, and belongs to the technical field of traditional Chinese medicines.

### BACKGROUND

Cough variant asthma (CVA) is also called cough-type asthma and has been called allergic bronchitis, allergic cough or occult asthma in the past. The CVA is mainly manifested by repeated and continuous dry cough with an itching feeling of throat, and sometimes a small amount of white phlegm difficult to cough out. The cough is usually paroxysmal and acute. The severe cough can be accompanied by symptoms of shortness of breath, loss of breath, low voice and short breath, etc. Some patients cough violently at night, which seriously affects sleep.

The CVA is characterized by chronic cough. Modern medicine considers that the cause is the stimulation of airways (such as smoke, cold air or allergens). Sometimes, cough is the only manifestation, without dyspnea and inertia. The treatment of the CVA is about the same as that of asthma. In clinical practice, traditional Chinese medicine often classify the CVA as "cough", "bronchial wheezing syndrome", "anemogenous cough", "lung obstruction", "pharyngo-genic cough" and also as "dyspnea syndrome". The cough and dyspnea symptoms can also be classified as "cough" and "endogenous cough" to carry out treatment based on syndrome differentiation. The disease is mostly caused by pathogen excessiveness and body resistance deficiency, and mixed deficiency and excessiveness. The pathogen excessiveness is mainly manifested by phlegm-dampness accumulating lung, phlegm-heat stagnating lung, and liver-fire attacking lung. The body resistance deficiency is mainly manifested by lung yin deficiency, lung-spleen deficiency, and lung-kidney deficiency. In the treatment, attention should be paid to strengthening body resistance and tonifying deficiency while eliminating pathogen to relieve cough, and three organs of the lungs, spleen, and kidneys.

Since the mechanism and cause of the CVA are not known exactly, most scholars think that the mechanism is consistent with that of typical asthma, that is, an airway allergic inflammation. Therefore, the current treatment is basically symptomatic. Common treatments for the CVA include environmental prophylaxis and hormonal therapy. The CVA is controlled by western medicines in the acute stage and treated by combination of traditional Chinese medicines and western medicines in the remission stage.

The common traditional Chinese medicine syndrome differentiation classification mode for cough symptoms is as follows:
(1) Wind-cold affecting lung: cough with loud voice, thin and white phlegm, aversion to cold, fever, no sweating, thin and white tongue coating, and superficial and tense pulse.
(2) Wind-heat attacking lung: cough with rough breath, sticky white or yellow phlegm, sore throat, cough with hoarseness, fever, slight aversion to wind-cold, slight thirst, red tongue tip, thin white or yellow coating, and superficial and rapid pulse.
(3) Dryness-evil impairing lung: dry cough with less phlegm, uncomfortable expectoration, dry nasopharynx, dry mouth, red tongue tip, thin and yellow coating with little fluid, and thready and rapid pulse.
(4) Phlegm-heat obstructing lung: cough with rough breath, excessive, thick and yellow phlegm, dysphoria with smothery sensation, dry mouth, red tongue, yellow and greasy coating, and slippery and rapid pulse.
(5) Liver-fire attacking lung: cough with reversed discharge of choking qi may cause pain in the chest and hypochondrium, hemoptysis, red tongue with thin, yellow and dry coating and wiry and rapid pulse.
(6) Phlegm-dampness accumulating lung: cough with heavy and turbid noise, excessive and white phlegm, heavy syndrome in the morning, chest stuffiness and stomach fullness, and poor appetite, white and greasy coating, and slippery pulse.
(7) Lung yin deficiency: prolonged cough with less phlegm, uncomfortable expectoration, sticky phlegm or phlegm with blood streak, dry throat and mouth, feverishness in palms and soles, red tongue with little coating, and thready and rapid pulse.
(8) Lung qi deficiency: chronic disease with low cough noise, cough with dyspnea, thin and white phlegm, poor appetite, short breath, chest stuffiness, tiredness, hypodynamia, spontaneous sweating, aversion to cold, pale and tender tongue with white coating, and weak pulse.

Yan Deyi (Yang Deyi, Treating 57 Cases of Cough Variant Asthma with Herba ephedrae and Radix Ephedrae Combined Herba Schizonepetae and Radix Saposhnikoviae Toxin-Vanquishing Powder, [J]. Jiangxi Journal of Traditional Chinese Medicine, 2006(7):32) used a *herba ephedrae* and *radix ephedrae* combined *herba schizonepetae* and *radix saposhnikoviae* toxin-vanquishing powder for modified treatment of 57 cases of cough variant asthma and the powder had an obvious curative effect. Ephedrine, a main active ingredient of *herba ephedrae,* can act on a plurality of systems of the body and can cause serious side effects on the cardiovascular and central nervous systems (Wang Xin, Xiao Nong, and Zhou Jiangbao, Side Effects of Ephedrine and Related Research Status, [J]. Foreign Medicine: Fascicule of Traditional Chinese Medicine, 2005, 27(3): 155-156). Liu Xiaoshuai et al (Liu Xiaoshuai, Zeng Nan, Liang Ke, Zhao Lu, Qu Liping, Song Meifang, and Zhang Chongyan, Experiment Research on Anti-Inflammatory Mechanism of Nitric Oxide Synthase-Nitric Oxide Pathway Intervened by Herba Schizonepetae and Radix Saposhnikoviae Powder, [J]. Lishizhen Medicine and Materia Medica Research, 2008,19(12): 3014-3015) used a mouse model of asthma induced by ovalbumin and a rat model of pleurisy induced by carrageenan, observed the activity of NOS and iNOS and the NO content in a pleural effusion of rats and a lung tissue homogenate of mice after the intervention with a *herba schizonepetae* and *radix saposhnikoviae* powder, and discussed the anti-inflammatory mechanism of the *herba schizonepetae* and *radix saposhnikoviae* powder. A *herba schizonepetae* and *radix saposhnikoviae* granule is prepared by mutually matching *herba schizonepetae, radix saposhnikoviae,* notopterygium root, *radix angelicae pubescentis, radix bupleuri, rhizoma chuanxiong, fructus aurantii,* poria cocos, *radix platycodonis,* liquorice root and the like, has the effects of inducing sweat and relieving exterior syndrome, dispelling wind and eliminating phlegm, and clearing lungs and relieving cough, and has very good effects on a series of symptoms after used for treating exogenous wind-cold. The cough variant asthma can be induced or aggravated after being infected by wind-cold. New indications in the aspect are found in the practical clinical application process, particularly, the cough is distinguished as cold cough with effects of wind-cold affecting lung, phlegm-dampness accumulating lung and the like. The clinical experience provides a support for further development and utilization of the product. At present, there is no study report on use of a formula of a *herba schizonepetae* and *radix saposhnikoviae* granule in cough variant asthma.

### SUMMARY

### Technical problem

The main purpose of the present invention is to provide a traditional Chinese medicinal composition. The traditional Chinese medicinal composition is prepared from notopterygium root, *radix angelicae pubescentis,* poria cocos, *radix saposhnikoviae, herba schizonepetae, rhizoma chuanxiong, radix platycodonis, radix bupleuri,* common hogfennel root, *fructus aurantii,* and liquorice root.

### Solution to problem

### Technical solution

The present invention further develops use of the traditional Chinese medicinal composition on the basis of the existing *herba schizonepetae* and *radix saposhnikoviae* granule product.

The inventor carries out research on the *herba schizonepetae* and *radix saposhnikoviae* granule for treating cough variant asthma, and finds that the *herba schizonepetae* and *radix saposhnikoviae* granule has a remarkable effect when used in the cough variant asthma and increases the medication selectivity of a patient. A formula of the *herba schizonepetae* and *radix saposhnikoviae* granule used in the present invention does not contain *herba ephedrae* and *radix ephedrae,* thereby reducing side effects brought by the *herba ephedrae* and *radix ephedrae,* and reducing a safety risk.

A first purpose of the present invention is to provide a traditional Chinese medicinal composition mainly prepared from *herba schizonepetae, radix saposhnikoviae,* notopterygium root, *radix angelicae pubescentis, radix bupleuri, rhizoma chuanxiong, fructus aurantii*, poria cocos, *radix platycodonis,* and liquorice root. That is the traditional Chinese medicinal composition is mainly prepared from the following raw materials in parts by weight:
5-30 parts of *herba schizonepetae,* 5-30 parts of *radix saposhnikoviae,* 5-30 parts of notopterygium root,
5-30 parts of *radix angelicae pubescentis,* 3-25 parts of *radix bupleuri,* 3-25 parts of common hogfennel root,
5-30 parts of *rhizoma chuanxiong,* 3-25 parts of *fructus aurantii,* 5-30 parts of poria cocos,
3-25 parts of *radix platycodonis,* and 1-10 parts of liquorice root.

Preferably, the traditional Chinese medicinal composition is mainly prepared from the following raw materials in parts by weight:
10-20 parts of *herba schizonepetae,* 10-20 parts of *radix saposhnikoviae,* 10-20 parts of notopterygium root,
10-20 parts of *radix angelicae pubescentis,* 3-20 parts of *radix bupleuri,* 3-20 parts of common hogfennel root,
10-20 parts of *rhizoma chuanxiong,* 3-20 parts of *fructus aurantii,* 10-25 parts of poria cocos,
3-20 parts of *radix platycodonis,* and 1-8 parts of liquorice root.

Further preferably, the traditional Chinese medicinal composition is mainly prepared from the following raw materials in parts by weight:
15 parts of *herba schizonepetae,* 15 parts of *radix saposhnikoviae,* 15 parts of notopterygium root,
15 parts of *radix angelicae pubescentis,* 15 parts of *radix bupleuri,* 15 parts of common hogfennel root,
15 parts of *rhizoma chuanxiong,* 15 parts *of fructus aurantii,* 15 parts of poria cocos,
15 parts of *radix platycodonis,* and 5 parts of liquorice root.

A second purpose of the present invention is to provide use of the traditional Chinese medicinal composition in the preparation of a drug for treating cough variant asthma.

The cough variant asthma of the present invention is mainly clinically manifested by cough, especially long-term intractable dry cough, and is often caused after the inhalation of a pungent odor (paint, gasoline, perfume, pollen and the like), cold air, allergen contact, strenuous exercise, and respiratory tract infection. Some patients have an onset of the asthma without any inducement. Variant asthma occurs mostly in the nighttime and early morning, mostly common in spring and autumn.

The clinical features of the cough variant asthma of the present invention are: over two months of causeless chronic cough with paroxysmal irritant dry cough or a small amount of white foamy phlegm; the disease can be aggravated by inhaling chemical odors such as smoke or paint, dichlorvos, etc.; and the application of various antibiotics is ineffective, and no obvious abnormality exists in X rays or CT examination. In 40% of patients, allergic rhinitis symptoms such as sneezing and running nose may be combined. Many foreign doctors refer to it as allergic rhinitis-bronchitis.

The cough variant asthma of the present invention is classified according to the syndrome types of traditional Chinese medicine. The syndrome types of traditional Chinese medicine of the cough variant asthma are cold wheezing, heat wheezing, exogenous cold and endogenous heat, and lung excess and kidney deficiency.

Cough variant asthma (CVA) is also called cough-type asthma and has been called allergic bronchitis, allergic cough or occult asthma in the past. The CVA is mainly manifested by repeated and continuous dry cough with an itching feeling of throat, and sometimes a small amount of white phlegm difficult to cough out. The cough is usually paroxysmal and acute. The severe cough can be accompanied by symptoms of shortness of breath, loss of breath, low voice and short breath, etc.

A third purpose of the present invention is to provide a method for preparing the traditional Chinese medicinal composition. The preparation method mainly comprises the following steps:
step A: distilling *herba schizonepetae, radix saposhnikoviae,* notopterygium root, *radix angelicae pubescentis,* common hogfennel root, *rhizoma chuanxiong,* and *fructus aurantii* to extract volatile oil for a standby application, and collecting distilled medicinal residues, and distilled *rhizoma chuanxiong* and *fructus aurantii* water solutions for a standby application;
step B: preparing the distilled *rhizoma chuanxiong* and *fructus aurantii* water solutions obtained in step A into a 10%-40% ethanol solution for a standby application;
step C: mixing poria cocos, and the distilled *rhizoma chuanxiong* and *fructus aurantii* medicinal residues obtained in step A, percolating same with the ethanol solution obtained in step B for extraction, and collecting a percolate for a standby application;
step D: decocting radix bupleuri, radix platycodonis, liquorice root, and the distilled *herba schizonepetae, radix saposhnikoviae,* notopterygium root, *radix angelicae pubescentis,* and common hogfennel root medicinal residues obtained in step A in water, and concentrating a decoction for a standby application; and
step E: mixing the percolate obtained in step C and the decoction obtained in step D, concentrating same, and adding the volatile oil obtained in step A to obtain the traditional Chinese medicinal composition.

Preferably, the preparation method mainly comprises the following steps:
step A: distilling *herba schizonepetae, radix saposhnikoviae,* notopterygium root, *radix angelicae pubescentis,* common hogfennel root, *rhizoma chuanxiong,* and *fructus aurantii* to extract volatile oil for a standby application, and collecting distilled medicinal residues, and distilled *rhizoma chuanxiong* and *fructus aurantii* water solutions for a standby application;
step B: preparing the distilled *rhizoma chuanxiong* and *fructus aurantii* water solutions obtained in step A into a 15%-30% ethanol solution for a standby application;
step C: mixing poria cocos, and the distilled *rhizoma chuanxiong* and *fructus aurantii* medicinal residues obtained in step A, percolating same with the ethanol solution obtained in step B for extraction, and collecting a percolate for a standby application;
step D: decocting radix bupleuri, radix platycodonis, liquorice root, and the distilled *herba schizonepetae, radix saposhnikoviae,* notopterygium root, *radix angelicae pubescentis,* and common hogfennel root medicinal residues obtained in step A in water, and concentrating a decoction into a thick paste for a standby application; and
step E: mixing the percolate obtained in step C and the thick paste obtained in step D, concentrating the mixture into a clear paste, and adding the volatile oil obtained in step A to obtain the traditional Chinese medicinal composition.

Further preferably, the preparation method mainly comprises the following steps:
step A: distilling *herba schizonepetae, radix saposhnikoviae,* notopterygium root, *radix angelicae pubescentis,* common hogfennel root, *rhizoma chuanxiong,* and *fructus aurantii* to extract volatile oil for a standby application, and collecting distilled medicinal residues, and distilled *rhizoma chuanxiong* and *fructus aurantii* water solutions for a standby application;
step B: preparing the distilled *rhizoma chuanxiong* and *fructus aurantii* water solutions obtained in step A into a 25% ethanol solution for a standby application;
step C: mixing poria cocos, and the distilled *rhizoma chuanxiong* and *fructus aurantii* medicinal residues obtained in step A, percolating same with the ethanol solution obtained in step B for extraction, and collecting a percolate for a standby application;
step D: decocting radix bupleuri, radix platycodonis, liquorice root, and the distilled *herba schizonepetae, radix saposhnikoviae,* notopterygium root, *radix angelicae pubescentis,* and common hogfennel root medicinal residues obtained in step A in water, and concentrating a decoction into a thick paste for a standby application; and
step E: mixing the percolate obtained in step C and the thick paste obtained in step D, concentrating the mixture into a clear paste, and adding the volatile oil obtained in step A to obtain the traditional Chinese medicinal composition.

A fourth purpose of the present invention is to provide a traditional Chinese medicine preparation containing the traditional Chinese medicinal composition, namely the traditional Chinese medicinal composition is prepared into a clinically acceptable dosage form directly by a conventional process or after adding a pharmaceutically acceptable auxiliary material.

Preferably, the clinically acceptable oral formulation is one or more of a pill, a capsule, a tablet, a granule or a liquid oral formulation.

Furthermore preferably, the oral formulation is a granule.

### Beneficial effects of invention

### Beneficial effects

Compared with the prior art, the present invention achieves the remarkable technical effects:

The traditional Chinese medicinal composition of the present invention can obviously reduce the cough frequency of a guinea pig model of cough variant asthma and reduce a percentage of eosinophils in a bronchoalveolar lavage fluid (BALF). Another example of the present invention shows that the traditional Chinese medicinal composition of the present invention can obviously reduce the cough frequency of a rat model of cough variant asthma and reduce a level of serum eosinophil cationic proteins. Eosinophil cationic proteins (ECPs) are a toxic protein released after EOS activation and can damage respiratory epithelium, and can cause allergic pathological changes such as airway spasm and mucosal edema, thereby causing high reactivity of the airways. The traditional Chinese medicinal composition of the present invention is safe in clinical medication and has a remarkable treatment effect on cough variant asthma.

### DETAILED DESCRIPTION OF THE EMBODIMENTS

### Examples of invention

In order to verify the efficacy of the traditional Chinese medicinal composition for cough variant asthma, the inventor carries out an animal experimental study. Only a part of experimental models are taken as examples for description. The inventor also carries out a pharmacological experimental study on other cough variant asthma recorded in the description. The composition of the present invention can achieve the same or similar effect, and the description is omitted herein.

The inventor explains that the following experimental studies are carried out on the basis of an acute toxicity test and a long-term toxicity test for proving the drug safety, and the administration dose in the experimental studies is within a safe dose range.

### Preparation example 1 Preparation of granule

### Prescription:

75 g of *herba schizonepetae,* 75g of *radix saposhnikoviae,* 75g of notopterygium root, 75 g of *radix angelicae pubescentis,* 75 g of *radix bupleuri,* 75 g of common hogfennel root,
75 g of *rhizoma chuanxiong,* 75 g of *fructus aurantii,* 75 g of poria cocos, 75 g of *radix platycodonis,* and 25 g of liquorice root.

### Preparation method:

step A: *herba schizonepetae, radix saposhnikoviae,* notopterygium root, *radix angelicae pubescentis,* common hogfennel root, *rhizoma chuanxiong,* and *fructus aurantii* were respectively distilled to extract volatile oil for a standby application, and distilled medicinal residues, and distilled *rhizoma chuanxiong* and *fructus aurantii* water solutions were collected for a standby application;
step B: the distilled *rhizoma chuanxiong* and *fructus aurantii* water solutions obtained in step A were prepared into a 25% ethanol solution for a standby application;
step C: mixing poria cocos, and the distilled *rhizoma chuanxiong* and *fructus aurantii* medicinal residues obtained in step A, percolating same with the ethanol solution obtained in step B for extraction, and collecting a percolate for a standby application;
step D: *radix bupleuri, radix platycodonis,* liquorice root, and the distilled *herba schizonepetae, radix saposhnikoviae,* notopterygium root, *radix angelicae pubescentis,* and common hogfennel root medicinal residues obtained in step A were decocted in water twice with 1.5 hours each time, two decoctions were mixed, the mixture was filtered, and a filtrate was concentrated into a thick paste for a standby application; and
step E: the percolate obtained in step C and the thick paste obtained in step D were mixed, the mixture was stood and filtered, a filtrate was concentrated into a clear paste, a proper amount of sucrose was added, the materials were uniformly mixed, the mixture was prepared into a granule, the granule was dried, the volatile oil obtained in step A was added, and the materials were uniformly mixed to obtain a granule.

### Preparation example 2 Preparation of granule

### Prescription:

10 g of *herba schizonepetae,* 10 g of *radix saposhnikoviae,* 12 g of notopterygium root, 12 g of *radix angelicae pubescentis,* 5 g of *radix bupleuri,* 5 g of common hogfennel root,
12 g of *rhizoma chuanxiong,* 5 g *of fructus aurantii,* 20 g of poria cocos, 5 g *of radix platycodonis,* and 3 g of liquorice root.

The preparation method was the same as that in example 1.

### Preparation example 3 Preparation of granule

### Prescription:

30 g of *herba schizonepetae,* 5 g of *radix saposhnikoviae,* 30 g of notopterygium root, 5 g of *radix angelicae pubescentis,* 25 g of *radix bupleuri,* 3 g of common hogfennel root,
30 g of *rhizoma chuanxiong,* 3 g *of fructus aurantii,* 30 g of poria cocos, 3 g *of radix platycodonis,* and 10 g of liquorice root.

The preparation method was the same as that in example 1.

### Preparation example 4 Preparation of oral liquid

### Prescription:

5 g of *herba schizonepetae,* 30 g of *radix saposhnikoviae,* 5 g of notopterygium root, 30 g of *radix angelicae pubescentis,* 3 g of *radix bupleuri,* 25 g of common hogfennel root,
5 g of *rhizoma chuanxiong,* 25 g *of fructus aurantii,* 5 g of poria cocos, 25 g *of radix platycodonis,* and 1 g of liquorice root.

### Preparation method:

step A: *herba schizonepetae, radix saposhnikoviae,* notopterygium root, *radix angelicae pubescentis,* common hogfennel root, *rhizoma chuanxiong,* and *fructus aurantii* were respectively distilled to extract volatile oil for a standby application, and distilled medicinal residues, and distilled *rhizoma chuanxiong* and *fructus aurantii* water solutions were collected for a standby application;
step B: the distilled *rhizoma chuanxiong* and *fructus aurantii* water solutions obtained in step A were prepared into a 10% ethanol solution for a standby application;
step C: mixing poria cocos, and the distilled *rhizoma chuanxiong* and *fructus aurantii* medicinal residues obtained in step A, percolating same with the ethanol solution obtained in step B for extraction, and collecting a percolate for a standby application;
step D: *radix bupleuri, radix platycodonis,* liquorice root, and the distilled *herba schizonepetae, radix saposhnikoviae,* notopterygium root, *radix angelicae pubescentis,* and common hogfennel root medicinal residues obtained in step A were decocted in water twice with 1.5 hours each time, two decoctions were mixed, the mixture was filtered, and a filtrate was concentrated into a thick paste for a standby application; and
step E: the percolate obtained in step C and the thick paste obtained in step D were mixed, the mixture was stood and filtered, a filtrate was concentrated into a clear paste, a proper amount of sucrose was added, the materials were uniformly mixed, the volatile oil obtained in step A was added, the materials were uniformly mixed, and water was added to 1,000 ml to obtain an oral liquid.

### Preparation example 5 Preparation of syrup

### Prescription:

20 g of *herba schizonepetae,* 10 g of *radix saposhnikoviae,* 20 g of notopterygium root, 10 g of *radix angelicae pubescentis,* 20 g of *radix bupleuri,* 3 g of common hogfennel root,
20 g of *rhizoma chuanxiong,* 3 g *of fructus aurantii,* 25 g of poria cocos, 3 g *of radix platycodonis,* and 8 g of liquorice root.

### Preparation method:

step A: *herba schizonepetae, radix saposhnikoviae,* notopterygium root, *radix angelicae pubescentis,* common hogfennel root, *rhizoma chuanxiong,* and *fructus aurantii* were respectively distilled to extract volatile oil for a standby application, and distilled medicinal residues, and distilled *rhizoma chuanxiong* and *fructus aurantii* water solutions were collected for a standby application;
step B: the distilled *rhizoma chuanxiong* and *fructus aurantii* water solutions obtained in step A were prepared into a 40% ethanol solution for a standby application;
step C: mixing poria cocos, and the distilled *rhizoma chuanxiong* and *fructus aurantii* medicinal residues obtained in step A, percolating same with the ethanol solution obtained in step B for extraction, and collecting a percolate for a standby application;
step D: *radix bupleuri, radix platycodonis,* liquorice root, and the distilled *herba schizonepetae, radix saposhnikoviae,* notopterygium root, *radix angelicae pubescentis,* and common hogfennel root medicinal residues obtained in step A were decocted in water twice with 1.5 hours each time, two decoctions were mixed, the mixture was filtered, and a filtrate was concentrated into a thick paste for a standby application; and
step E: the percolate obtained in step C and the thick paste obtained in step D were mixed, the mixture was stood and filtered, a filtrate was concentrated into a clear paste, a proper amount of sucrose was added, the materials were uniformly mixed, the volatile oil obtained in step A and 500 ml of simple syrup were added, the materials were uniformly mixed, the mixture was stood and filtered, and water was added to 1,000 ml to obtain a syrup.

### Preparation example 6 Preparation of tablet

### Prescription:

10 g of *herba schizonepetae,* 20 g of *radix saposhnikoviae,* 10 g of notopterygium root, 20 g of *radix angelicae pubescentis,* 3 g of *radix bupleuri,* 20 g of common hogfennel root,
10 g of *rhizoma chuanxiong,* 20 g of *fructus aurantii,* 10 g of poria cocos, 20 g of *radix platycodonis,* and 1 g of liquorice root.

### Preparation method:

step A: *herba schizonepetae, radix saposhnikoviae,* notopterygium root, *radix angelicae pubescentis,* common hogfennel root, *rhizoma chuanxiong,* and *fructus aurantii* were respectively distilled to extract volatile oil for a standby application, and distilled medicinal residues, and distilled *rhizoma chuanxiong* and *fructus aurantii* water solutions were collected for a standby application;
step B: the distilled *rhizoma chuanxiong* and *fructus aurantii* water solutions obtained in step A were prepared into a 15% ethanol solution for a standby application;
step C: mixing poria cocos, and the distilled *rhizoma chuanxiong* and *fructus aurantii* medicinal residues obtained in step A, percolating same with the ethanol solution obtained in step B for extraction, and collecting a percolate for a standby application;
step D: *radix bupleuri, radix platycodonis,* liquorice root, and the distilled *herba schizonepetae, radix saposhnikoviae,* notopterygium root, *radix angelicae pubescentis,* and common hogfennel root medicinal residues obtained in step A were decocted in water twice with 1.5 hours each time, two decoctions were mixed, the mixture was filtered, and a filtrate was concentrated into a thick paste for a standby application; and
step E: the percolate obtained in step C and the thick paste obtained in step D were mixed, the mixture was stood and filtered, a filtrate was concentrated into a clear paste, a proper amount of sucrose was added, the materials were uniformly mixed, the mixture was prepared into a granule, the granule was dried, the volatile oil obtained in step A was added, the mixture was prepared into a granule, a proper amount of an excipient was added, the materials were uniformly mixed, and the mixture was tableted to obtain a tablet.

### Preparation example 7 Preparation of capsule

### Prescription:

20 g of *herba schizonepetae,* 10 g of *radix saposhnikoviae,* 20 g of notopterygium root, 10 g of *radix angelicae pubescentis,* 20 g of *radix bupleuri,* 3 g of common hogfennel root,
20 g of *rhizoma chuanxiong,* 3 g *of fructus aurantii,* 25 g of poria cocos, 3 g *of radix platycodonis,* and 8 g of liquorice root.

step A: *herba schizonepetae, radix saposhnikoviae,* notopterygium root, *radix angelicae pubescentis,* common hogfennel root, *rhizoma chuanxiong,* and *fructus aurantii* were respectively distilled to extract volatile oil for a standby application, and distilled medicinal residues, and distilled *rhizoma chuanxiong* and *fructus aurantii* water solutions were collected for a standby application;
step B: the distilled *rhizoma chuanxiong* and *fructus aurantii* water solutions obtained in step A were prepared into a 30% ethanol solution for a standby application;
step C: mixing poria cocos, and the distilled *rhizoma chuanxiong* and *fructus aurantii* medicinal residues obtained in step A, percolating same with the ethanol solution obtained in step B for extraction, and collecting a percolate for a standby application;
step D: *radix bupleuri, radix platycodonis,* liquorice root, and the distilled *herba schizonepetae, radix saposhnikoviae,* notopterygium root, *radix angelicae pubescentis,* and common hogfennel root medicinal residues obtained in step A were decocted in water twice with 1.5 hours each time, two decoctions were mixed, the mixture was filtered, and a filtrate was concentrated into a thick paste for a standby application; and
step E: the percolate obtained in step C and the thick paste obtained in step D were mixed, the mixture was stood and filtered, a filtrate was concentrated into a clear paste, a proper amount of sucrose was added, the materials were uniformly mixed, the mixture was prepared into a granule, the granule was dried, the volatile oil obtained in step A was added, the materials were uniformly mixed, the mixture was prepared into a granule, the granule was dried and crushed, and the crushed granule was encapsulated to obtain a capsule.

### Preparation example 8 Preparation of pill

### Prescription:

10 g of *herba schizonepetae,* 20 g of *radix saposhnikoviae,* 10 g of notopterygium root, 20 g of *radix angelicae pubescentis,* 3 g of *radix bupleuri,* 20 g of common hogfennel root,
10 g of *rhizoma chuanxiong,* 20 g of *fructus aurantii,* 10 g of poria cocos, 20 g of *radix platycodonis,* and 1g of liquorice root.

### Preparation method:

step A: *herba schizonepetae, radix saposhnikoviae,* notopterygium root, *radix angelicae pubescentis,* common hogfennel root, *rhizoma chuanxiong,* and *fructus aurantii* were respectively distilled to extract volatile oil for a standby application, and distilled medicinal residues, and distilled *rhizoma chuanxiong* and *fructus aurantii* water solutions were collected for a standby application;
step B: the distilled *rhizoma chuanxiong* and *fructus aurantii* water solutions obtained in step A were prepared into a 25% ethanol solution for a standby application;
step C: mixing poria cocos, and the distilled *rhizoma chuanxiong* and *fructus aurantii* medicinal residues obtained in step A, percolating same with the ethanol solution obtained in step B for extraction, and collecting a percolate for a standby application;
step D: *radix bupleuri, radix platycodonis,* liquorice root, and the distilled *herba schizonepetae, radix saposhnikoviae,* notopterygium root, *radix angelicae pubescentis,* and common hogfennel root medicinal residues obtained in step A were decocted in water twice with 1.5 hours each time, two decoctions were mixed, the mixture was filtered, and a filtrate was concentrated into a thick paste for a standby application; and
step E: the percolate obtained in step C and the thick paste obtained in step D were mixed, the mixture was stood and filtered, a filtrate was concentrated into a clear paste, a proper amount of sucrose was added, the materials were uniformly mixed, the mixture was prepared into a granule, the granule was dried, the volatile oil obtained in step A was added, the materials were uniformly mixed, the mixture was dried, crushed and sieved, 40-60 g of refined honey and a proper amount of water were added to prepare a pill, and the pill was dried to obtain a finished product.

### Preparation example 9 Preparation of extract

### Prescription:

75 g of *herba schizonepetae,* 75g of *radix saposhnikoviae,* 75g of notopterygium root, 75 g of *radix angelicae pubescentis,* 75 g of *radix bupleuri,* 75 g of common hogfennel root,
75 g of *rhizoma chuanxiong,* 75 g of *fructus aurantii,* 75 g of poria cocos, 75 g of *radix platycodonis,* and 25 g of liquorice root.

### Preparation method:

step A: *herba schizonepetae, radix saposhnikoviae,* notopterygium root, *radix angelicae pubescentis,* common hogfennel root, *rhizoma chuanxiong,* and *fructus aurantii* were respectively distilled to extract volatile oil for a standby application, and distilled medicinal residues, and distilled *rhizoma chuanxiong* and *fructus aurantii* water solutions were collected for a standby application;
step B: the distilled *rhizoma chuanxiong* and *fructus aurantii* water solutions obtained in step A were prepared into a 25% ethanol solution for a standby application;
step C: mixing poria cocos, and the distilled *rhizoma chuanxiong* and *fructus aurantii* medicinal residues obtained in step A, percolating same with the ethanol solution obtained in step B for extraction, and collecting a percolate for a standby application;
step D: *radix bupleuri, radix platycodonis,* liquorice root, and the distilled *herba schizonepetae, radix saposhnikoviae,* notopterygium root, *radix angelicae pubescentis,* and common hogfennel root medicinal residues obtained in step A were decocted in water twice with 1.5 hours each time, two decoctions were mixed, the mixture was filtered, and a filtrate was concentrated into a thick paste for a standby application; and
step E: the percolate obtained in step C and the thick paste obtained in step D were mixed, the mixture was stood and filtered, a filtrate was concentrated into a clear paste, the volatile oil obtained in step A was added, and the materials were uniformly mixed to obtain an extract.

### Industrial applicability

### Pharmacodynamic example I Effect of traditional Chinese medicinal composition of the present invention on guinea pig model of cough variant asthma

### 1 Materials

### 1.1 Animals

60 conventional guinea pigs, half male and female, aged 60-90 days, with a body mass of (300±30) g were used. The experimental animals were provided by the New Medicine Pharmacological Center of the Lunan Pharmaceutical Group Corporation, and were adaptively fed for one week before an experiment.

### 1.2 Drugs and reagents

### 1.2.1 Drugs

### Granule of example 1;

*herba ephedrae* and *radix ephedrae* combined *herba schizonepetae* and *radix saposhnikoviae* toxin-vanquishing powder with the formula and proportion in the literature (Yang Deyi, Treating 57 Cases of Cough Variant Asthma with Herba ephedrae and Radix Ephedrae Combined Herba Schizonepetae and Radix Saposhnikoviae Toxin-Vanquishing Powder, [J]. Jiangxi Journal of Traditional Chinese Medicine, 2006(7):32-32);
chicken ovalbumin (OVA) (GBCBIO Technologies, batch No. A20199006-59);
aluminum hydroxide dry powder (Tianjin Huirui Chemical Technology Co., Ltd., batch No. 20201209); and
capsaicin (Sigma, USA, batch No. 20200401).

### 1.2.2 Dose in guinea pigs

Granule of example 1: 3.6 g/kg high dose, 1.8 g/kg medium dose, and 0.9 g/kg low dose; and
*herba ephedrae* and *radix ephedrae* combined *herba schizonepetae* and *radix saposhnikoviae* toxin-vanquishing powder: 1.8 g/kg.

### 1.3 Primary reagents

Hematoxylin and eosin purchased from Boster Bio (Wuhan).

### 2. Grouping, modelling and administration

### 2.1 Grouping

After adaptively fed for 1 week, the animals were randomly divided into a normal control group, a model control group, a *herba ephedrae* and *radix ephedrae* combined *herba schizonepetae* and *radix saposhnikoviae* toxin-vanquishing powder group, and example 1 granule low-, medium- and high-dose groups according to the body mass, with 10 animals in each group.

### 2.2 Modelling

The experimental animals were randomly divided into a normal control group, a model control group, example 1 granule low-, medium- and high-dose groups, and a *herba ephedrae* and *radix ephedrae* combined *herba schizonepetae* and *radix saposhnikoviae* toxin-vanquishing powder group.

The animals in the modelling groups were smoked half an hour every day on the 1st to 28th days; on the 15th day, each guinea pig was injected with a suspension of 1 mL of 20 g/L OVA and 200 mg of aluminum hydroxide; on the 22nd day, each guinea pig was sensitized 1 time with a suspension of 1 mL of 20 g/L OVA and 200 mg of aluminum hydroxide in an enhanced manner; on the 29th day, each guinea pig was subjected to atomization attack by 10 g/L OVA continuously for 7 days with 1 time per day; and the guinea pigs in the normal control group were given the same volume of a normal saline.

All the animals were fed in normal environment with 12h/12h light cycle, and had free access to water and a pellet feed. Smoking box and use method: a smoking box was a customized stainless steel box body with the length, width and height of 100 cm×60 cm×60 cm respectively. A cigarette used in this study was the "red plum" filter cigarette (Hongta Tobacco (Group) Co., Ltd.; and flue-cured type cigarette, the tar content of 10 mg, the nicotine content in smoke of 0.7 mg, and the carbon monoxide content in smoke of 12 mg). After 10 cigarettes were lit in the smoking box each time, the smoke was discharged into the smoking box manually via a 300 mL syringe and a t-branch pipe until the cigarettes were burned out.

### 2.3 Intervention

The administration began on the 29th day, the day of atomization attack. The guinea pigs in each administration group were gavaged with 10 ml/kg of the corresponding drug 1 time per day continuously for 7 days. The guinea pigs in the normal control group and the model control group were given the same volume of purified water.

### 2.4 Specimen collection and detection

### 2.4.1 Determination of cough reflex sensitivity (CRS)

### 2.4.2 Percentage of eosinophils in bronchoalveolar lavage fluid

### 2.5 Statistical methods

An analysis was performed using a SPSS19.0 statistical software. The experimental data were expressed by *x̅* ± **s**, a variance analysis was performed for a comparison among groups after a homogeneity test of variance, and an LSD method was used for a pairwise comparison. AP < 0.05 indicated that the difference was statistically significant.

### 3. Examination items

### 3.1 Determination of CRS in each group of guinea pigs

The CRS in the airways was determined by a capsaicin cough provocation test. A whole body plethysmography (Buxco Electronics) was used. On the 2nd day after the last atomization attack with 10 g/L OVA, the guinea pigs were put into a plethysmography chamber and the cough frequency in 5 min was observed and recorded after 2 min of atomization with 10⁻⁴ mol/L of a capsaicin solution.

### 3.2 Determination of percentage of eosinophils in bronchoalveolar lavage fluid in each group of guinea pigs

After completion of the provocation test, pulmonary alveoli were lavaged 3 times with a phosphate buffered saline (PBS) with 2 ml each time, and a bronchoalveolar lavage fluid (BALF) was recovered. 200 µL of the collected BALF was taken and centrifuged at 3,000 r/min for 10 min, a supernatant was transferred, an erythrocyte lysate was added, the mixture was centrifuged at 3,000 r/min for 10 min, a supernatant was transferred, an equivalent amount of PBS was added, and smear and staining with hematoxylin-eosin (HE) were performed. The number of eosinophils per 200 cells in a continuous field was counted under a microscope, and a percentage of eosinophils was calculated.

### 4. Results and conclusions

### 4.1 Determination of CRS in each group of guinea pigs

The results in table 1 showed that: the cough frequency of the model control group was obviously increased with a statistically significant difference (P < 0.05) compared with that of the normal control group;
the cough frequency of the treatment groups was significantly lower than that of the model control group, namely, the cough frequencies of the example 1 granule low-, medium-and high-dose groups were significantly reduced with a statistically significant difference (*P* < 0.05 or *P* < 0.01) compared with that of the model control group; and
the cough frequency of the example 1 granule high-dose group was obviously reduced with a statistically significant difference (*P* < 0.05) compared with that of the *herba ephedrae* and *radix ephedrae* combined *herba schizonepetae* and *radix saposhnikoviae* toxin-vanquishing powder group.

The experimental results showed that the *herba schizonepetae* and *radix saposhnikoviae* granule can inhibit the cough reaction of guinea pigs and relieve cough. The effect of relieving cough had a certain relation with the dose of the granule.

**Table 1 Comparison of cough frequency in each group of guinea pigs (x̅ ± s, n = 10)**

| Groups | | Cough frequency (times/min) |
|---|---|---|
| Normal control group | | 0.78±0.42 |
| Model control group | | 4.24±0.72^{##} |
| *Herba ephedrae* and *radix ephedrae* combined *herba schizonepetae* and *radix saposhnikoviae* toxin-vanquishing powder group | | 2.96±0.68* |
| Example 1 granule groups | High-dosage group | 1.24±0.76***^{Δ}* |
| | Medium-dosage group | 1.86±0.64** |
| | Low-dosage group | 2.25±0.81** |

| | | |
|---|---|---|
| Note: compared with the normal control group, ^{#}*P* < 0.05 and ^{##}*P* < 0.01; compared with the model control group,**P* < 0.05 and ***P* < 0.01; and compared with the *herba ephedrae* and *radix ephedrae* combined *herba schizonepetae* and *radix saposhnikoviae* toxin-vanquishing powder group, ^{Δ}*P* < 0.05 and ^{ΔΔ}*P* < 0.01. | | |

### 4.2 Determination of percentage of eosinophils in bronchoalveolar lavage fluid in each group of guinea pigs

The results in table 2 showed that: the percentage of eosinophils of the model control group was obviously increased with a statistically significant difference (*P* < 0.05) compared with that of the normal control group;
the percentages of eosinophils in the 4 treatment groups were remarkably reduced all with a statistically significant difference (*P* < 0.05 or *P* < 0.01) compared with that of the model control group; and
the percentage of eosinophils in the example 1 granule high-dose group was obviously reduced with a statistically significant difference (P < 0.05) compared with that of the *herba ephedrae* and *radix ephedrae* combined *herba schizonepetae* and *radix saposhnikoviae* toxin-vanquishing powder group.

**Table 2 Comparison of percentage of eosinophils in BALF in each group of guinea pigs ( x̅ ± s, n = 10)**

| Groups | | Percentage of eosinophils |
|---|---|---|
| Normal control group | | 0.05±0.01 |
| Model control group | | 0.36±0.04^{##} |
| *Herba ephedrae* and *radix ephedrae* combined *herba schizonepetae* and *radix saposhnikoviae* toxin-vanquishing powder group | | 0.21±0.09 * |
| Example 1 granule groups | High-dosage group | 0.11±0.06***^{Δ}* |
| | Medium-dosage group | 0.14±0.05** |
| | Low-dosage group | 0.16±0.08** |

| | | |
|---|---|---|
| Note: compared with the normal control group, ^{#}*P* < 0.05 and ^{##}*P* < 0.01; compared with the model control group,**P* < 0.05 and ***P* < 0.01; and compared with the *herba ephedrae* and *radix ephedrae* combined *herba schizonepetae* and *radix saposhnikoviae* toxin-vanquishing powder group, ^{Δ}*P* < 0.05 and ^{ΔΔ}*P* < 0.01. | | |

In conclusion, the traditional Chinese medicinal composition of the present invention can obviously reduce the cough frequency of a guinea pig model of CVA and reduce a percentage of eosinophils in a bronchoalveolar lavage fluid (BALF). The experimental results showed that the traditional Chinese medicinal composition of the present invention can achieve the purpose of treating cough variant asthma.

Pharmacodynamic example II Effect of traditional Chinese medicinal composition of the present invention on rats with cough variant asthma

### 1 Materials

### 1.1 Animals

6-week old male clean-grade SD rats with a body mass of (220±20) g were used. The experimental animals were provided by the New Medicine Pharmacological Center of the Lunan Pharmaceutical Group Corporation, and were adaptively fed for one week before an experiment.

### 1.2 Drugs

### Granule of example 1;

*herba schizonepetae* and *radix saposhnikoviae* powder prepared from the components and by the method according to the literature (Liu Xiaoshuai, Zeng Nan, Liang Ke, Zhao Lu, Qu Liping, Song Meifang, and Zhang Chongyan, Experiment Research on Anti-Inflammatory Mechanism of Nitric Oxide Synthase-Nitric Oxide Pathway Intervened by Herba Schizonepetae and Radix Saposhnikoviae Powder, [J]. Lishizhen Medicine and Materia Medica Research, 2008,19(12): 3014-3015);
chicken ovalbumin (OVA) (GBCBIO Technologies, batch No. A20199006-59);
aluminum hydroxide dry powder (Tianjin Huirui Chemical Technology Co., Ltd., batch No. 20201209); and
capsaicin (Sigma, USA, batch No. 20200401).

### 1.2.2 Dose in rats

Granule of example 1: 3.6 g/kg high dose, 1.8 g/kg medium dose, and 0.9 g/kg low dose; and
*herba schizonepetae* and *radix saposhnikoviae* powder: 2.5 g/kg.

### 2. Grouping, modelling and administration

Modelling was performed by using a method of jointly provocating sensitization by ovalbumin and aluminum hydroxide. Except for 8 rats in a normal control group, the rest rats were injected subcutaneously with 1 mL of a prepared normal saline containing 1 mg of ovalbumin and 10 mg of aluminum hydroxide. A total of 10 spots were taken on the two hind foot soles, groin, waist, back and neck respectively, and each spot was injected subcutaneously with 0.05 mL of the normal saline, and the rats were simultaneously intraperitoneally injected with 0.5 mL of the prepared normal saline, 1 mL in total. The injection was repeated once on the 8th day to enhance sensitization. From the 15th day, the rats were placed in a sealed organic glass cover and subjected to ultrasonic atomization provocation for 20 min by giving a normal saline containing a 1% ovalbumin solution to provocate asthma. An obvious contraction of the abdominal muscle of the rats was regarded to be positive and the modelling was successful until respiratory tract spasm symptoms such as deepened and accelerated breathing appeared.

The successfully modelled rats were randomly divided into 5 groups (a model control group, example 1 granule high-, medium-, and low-dose groups, and a *herba schizonepetae* and *radix saposhnikoviae* powder group), with 8 rats in each group. The doses of the example 1 granule low-, medium-, and high-dose groups were 0.9 g/kg, 1.8 g/kg and 3.6 g/kg respectively, and the dose of the *herba schizonepetae* and *radix saposhnikoviae* powder group was 2.5 g/kg. The drug was dissolved in purified water and the administration volume was 10 mL/kg. The rats in the normal control group and the model control group were gavaged with an equal volume of purified water. The administration was continued for 13 days.

### 3. Indicator detection

### 3.1 Determination of cough frequency

### 3.2 Determination of content of serum eosinophil cationic proteins (ECPs)

### 3.3 Statistical methods

An analysis was performed using a SPSS19.0 statistical software. The experimental data were expressed by *x̅* ± *s*, a variance analysis was performed for a comparison among groups after a homogeneity test of variance, and an LSD method was used for a pairwise comparison. *A P* < 0.05 indicated that the difference was statistically significant.

### 4. Results

### 4.1 Comparison of determination results of cough frequency of rats in each group

On the 13rd day after the administration, the rats in each group were placed in a closed box and the cough frequency after atomization inhalation of a capsaicin solution at a concentration of 1×10⁻⁴ mol/L within 5 min was recorded and counted.

**Table 3 Comparison of cough frequency in each group of rats ( x̅ ± s, n = 8)**

| Groups | Cough frequency (times/min) |
|---|---|
| Normal control group | 0.65±0.28 |
| Model control group | 3.85±0.79^{##} |
| Example 1 granule low-dose group | 2.98±0.43* |
| Example 1 granule medium-dose group | 2.6±0.52**^{Δ} |
| Example 1 granule high-dose group | 2.10±0.49**^{ΔΔ} |
| *Herba schizonepetae* and *radix saposhnikoviae* powder group | 3.20±0.41 |

| | |
|---|---|
| Note: compared with the normal control group, ^{#}*P* < 0.05 and ^{##}*P* < 0.01; compared with the model control group,**P* < 0.05 and **P < 0.01; and compared with the *herba schizonepetae* and *radix saposhnikoviae* powder group, ^{Δ}*P* < 0.05 and ^{ΔΔ}*P* < 0.01. | |

The cough frequency of the model control group was obviously increased with a statistically significant difference (*P* < 0.01) compared with that of the normal control group; the cough frequencies of the example 1 granule low-, medium- and high-dose groups were obviously reduced with a statistically significant difference (*P* < 0.05 and *P* < 0.01) compared with that of the model control group; and the cough frequencies of the example 1 granule medium- and high-dose groups were obviously reduced with a statistically significant difference (*P* < 0.05 and *P* < 0.01) compared with that of the *herba schizonepetae* and *radix saposhnikoviae* powder group.

The experimental results showed that the *herba schizonepetae* and *radix saposhnikoviae* granule can inhibit the cough reaction of rats and had the effect of inhibiting cough variant asthma. The effect of relieving cough was superior to that of the *herba schizonepetae* and *radix saposhnikoviae* powder.

### 4.2 Comparison of content of ECP in serum of rats in each group

After the animals were sacrificed on the 14th day of the experiment, 5 mL of blood was taken from the abdominal aorta, stood at 4°C, and centrifuged at 2,000 r/min for 10 min. Serum was separated and stored at -20°C. The determination was performed strictly according to the instructions of the ECP ELISA kit.

**Table 4 Concentration levels of serum ECPs in rats of each group ( x̅ ± s, n = 8)**

| Groups | ECP (ng/mL) |
|---|---|
| Normal control group | 1.43±0.37 |
| Model control group | 2.41±0.46^{##} |
| Example 1 granule low-dose group | 2.03±0.34 |
| Example 1 granule medium-dose group | 1.79±0.24**^{Δ} |
| Example 1 granule high-dose group | 1.70±0.26**^{ΔΔ} |
| *Herba schizonepetae* and *radix saposhnikoviae* powder group | 2.06±0.21 |

| | |
|---|---|
| Note: compared with the normal control group, ^{#}*P* < 0.05 and ^{##}*P* < 0.01; compared with the model control group,**P* < 0.05 and **P < 0.01; and compared with the *herba schizonepetae* and *radix saposhnikoviae* powder group, ^{Δ}*P* < 0.05 and ^{ΔΔ}*P* < 0.01. | |

The serum ECP content of the model control group was obviously increased (*P* < 0.01) compared with that of the normal control group; after the treatment, the serum ECP content of the example 1 granule medium- and high-dose groups was obviously reduced (*P* < 0.05 and *P* < 0.01) compared with that of the model control group; and serum ECP-reducing effects of the example 1 granule medium- and high-dose groups were better than that of the *herba schizonepetae* and *radix saposhnikoviae* powder group (*P* < 0.05 and *P* < 0.01). The test results showed that the *herba schizonepetae* and *radix saposhnikoviae* granule can reduce the ECP level of the organism and relieve allergic symptoms of cough variant asthma.

## Claims

1. Use of a traditional Chinese medicinal composition comprising *herba schizonepetae, radix saposhnikoviae,* notopterygium root, *radix angelicae pubescentis, radix bupleuri,* common hogfennel root, *rhizoma chuanxiong, fructus aurantii,* poria cocos, *radix platycodonis,* and liquorice root in the preparation of a drug for treating cough variant asthma.

2. The use according to claim 1, wherein the cough variant asthma is any one type of cold wheezing, heat wheezing, exogenous cold and endogenous heat, and lung excess and kidney deficiency.

3. The use according to claim 1, wherein the traditional Chinese medicinal composition can reduce a percentage of eosinophils and a level of serum eosinophil cationic proteins in a bronchoalveolar lavage fluid (BALF) of a patient with cough variant asthma.

4. The use according to any one of claims 1-3, wherein the traditional Chinese medicinal composition is mainly prepared from the following raw materials in parts by weight:
5-30 parts of *herba schizonepetae,* 5-30 parts of *radix saposhnikoviae,* 5-30 parts of notopterygium root,
5-30 parts of *radix angelicae pubescentis,* 3-25 parts of *radix bupleuri,* 3-25 parts of common hogfennel root,
5-30 parts of *rhizoma chuanxiong,* 3-25 parts of *fructus aurantii,* 5-30 parts of poria cocos,
3-25 parts of *radix platycodonis,* and 1-10 parts of liquorice root.

5. The use according to claim 4, wherein the traditional Chinese medicinal composition is mainly prepared from the following raw materials in parts by weight:
10-20 parts of *herba schizonepetae,* 10-20 parts of *radix saposhnikoviae,* 10-20 parts of notopterygium root,
10-20 parts of *radix angelicae pubescentis,* 3-20 parts of *radix bupleuri,* 3-20 parts of common hogfennel root,
10-20 parts of *rhizoma chuanxiong,* 3-20 parts of *fructus aurantii,* 10-25 parts of poria cocos,
3-20 parts of *radix platycodonis,* and 1-8 parts of liquorice root.

6. The use according to claim 5, wherein the traditional Chinese medicinal composition is mainly prepared from the following raw materials in parts by weight:
15 parts of *herba schizonepetae,* 15 parts of *radix saposhnikoviae,* 15 parts of notopterygium root,
15 parts of *radix angelicae pubescentis,* 15 parts of *radix bupleuri,* 15 parts of common hogfennel root,
15 parts of *rhizoma chuanxiong,* 15 parts *of fructus aurantii,* 15 parts of poria cocos,
15 parts of *radix platycodonis,* and 5 parts of liquorice root.

7. The use according to any one of claims 1-6, wherein a method for preparing the traditional Chinese medicinal composition comprises the following steps:
step A: distilling *herba schizonepetae, radix saposhnikoviae,* notopterygium root, *radix angelicae pubescentis,* common hogfennel root, *rhizoma chuanxiong,* and *fructus aurantii* to extract volatile oil for a standby application, and collecting distilled medicinal residues, and distilled *rhizoma chuanxiong* and *fructus aurantii* water solutions for a standby application;
step B: preparing the distilled *rhizoma chuanxiong* and *fructus aurantii* water solutions obtained in step A into a 10%-40% ethanol solution for a standby application;
step C: mixing poria cocos, and the distilled *rhizoma chuanxiong* and *fructus aurantii* medicinal residues obtained in step A, percolating same with the ethanol solution obtained in step B for extraction, and collecting a percolate for a standby application;
step D: decocting radix bupleuri, radix platycodonis, liquorice root, and the distilled *herba schizonepetae, radix saposhnikoviae,* notopterygium root, *radix angelicae pubescentis,* and common hogfennel root medicinal residues obtained in step A in water, and concentrating a decoction for a standby application; and
step E: mixing the percolate obtained in step C and the decoction obtained in step D, concentrating same, and adding the volatile oil obtained in step A to obtain the traditional Chinese medicinal composition,
preferably, step B being preparing the distilled *rhizoma chuanxiong* and *fructus aurantii* water solutions obtained in step A into a 15%-30% ethanol solution for a standby application; and
further preferably, step B being preparing the distilled *rhizoma chuanxiong* and *fructus aurantii* water solutions obtained in step A into a 25% ethanol solution for a standby application.

8. The use according to claim 7, wherein the traditional Chinese medicinal composition does not contain or contains one or more pharmaceutically acceptable pharmaceutical excipients.

9. The use according to claim 8, wherein the traditional Chinese medicinal composition can be prepared into one or more of a tablet, an oral liquid, a capsule, a pill, a granule or a liquid oral formulation.

10. The use according to claim 9, wherein the oral formulation is a granule.
